# EUROPÄISCHE PATENTSCHRIFT

(11) **EP 0 879 605 B1**
(45) Veröffentlichungstag und Bekanntmachung des Hinweises auf die Patenterteilung: **26.07.2006**
(21) Anmeldenummer: 98108041.9
(22) Anmeldetag: 02.05.1998
(51) Int. Cl.: A61K 47/48

(54) **Glykosyl-prodrogue Konjugat mit erhöhter Verträglichkeit**
Glycosyl prodrug conjugate with enhanced tolerance
Glycosyl-prodrogue conjugue avec une tolerance amelioré

(30) Priorität: 15.05.1997 DE 19720312
(43) Veröffentlichungstag der Anmeldung: 25.11.1998
(73) Patentinhaber: Sanofi-Aventis Deutschland GmbH, 65929 Frankfurt am Main (DE)
(72) Erfinder: Bosslet, Klaus, Dr., Gaithersburg, MD 20879 (US); Czech, Jörg, Dr., 35041 Marburg (DE); Gerken, Manfred, Dr., 35041 Marburg (DE); Straub, Rainer, DI., 35039 Marburg (DE); Blumrich, Matthias, Dr., 35435 Wettenberg (DE)

(56) Entgegenhaltungen:
- EP-A- 0 511 917
- EP-A- 0 540 859
- EP-A- 0 595 133
- EP-A- 0 642 799
- EP-A- 0 751 144
- EP-A- 0 795 334
- LEENDERS R G G ET AL: "beta-Glucuronyl Carbamate Based Pro-moieties Designed for Prodrugs in ADEPT" TETRAHEDRON LETTERS, Bd. 36, Nr. 10, 6. März 1995, Seite 1701-1704 XP004028569
- BOSSLET K ET AL: "TUMOR-SELECTIVE PRODRUG ACTIVATION BY FUSION PROTEIN-MEDIATED CATALYSIS" CANCER RESEARCH, Bd. 54, Nr. 7, 15. April 1994, Seiten 2151-2159, XP002038655
- HOUBA PH ET AL: "Characterization of novel anthracycline prodrugs activated by human beta-glucuronidase for use in antibody-directed enzyme prodrug therapy." BIOCHEM PHARMACOL, AUG 9 1996, 52 (3) P455-63, XP002076227 ENGLAND
- GESSON JP ET AL: "Prodrugs of anthracyclines for chemotherapy via enzyme-monoclonal antibody conjugates." ANTICANCER DRUG DES, OCT 1994, 9 (5) P409-23, XP002076228 ENGLAND
- HAISMA HJ ET AL: "Construction and characterization of a fusion protein of single-chain anti-CD20 antibody and human beta-glucuronidase for antibody-directed enzyme prodrug therapy." BLOOD, JUL 1 1998, 92 (1) P184-90, XP002076505 UNITED STATES
- SINHABABU A.K. ET AL: "Prodrugs of anticancer agents" ADVANCED DRUG DELIVERY REVIEWS, 1996, 19/2 (241-273), XP002076230 Netherlands

## Beschreibung

Die Therapie bösartiger Tumore, entzündlicher Erkrankungen oder Autoimmunerkrankungen ist neben der unzureichenden Wirksamkeit der Therapeutika mit starken Nebenwirkungen verbunden. Dieser Mangel kann hauptsächlich mit der zu geringen in vivo Verträglichkeit der eingesetzten Wirkstoffe erklärt werden.

Die Erfindung bezweckt, durch Modifizierung der Zubereitung die Verträglichkeit von Wirkstoffen in der Therapie zu verbessern und gegebenenfalls die Wirksamkeit zu erhöhen.

Die Erfindung betrifft daher eine Zubereitung, enthaltend
1) eine Verbindung der Formel I,

   Glykosyl-Y[-C(=Y)-X-]ₚ-W(R)ₙ-X-C(=Y)-Wirkstoff (I)

   worin
   - Glykosyl: ein enzymatisch abspaltbares Poly-, Oligo- oder Monosaccharid ist
   - W: ein Phenylrest oder ein mehrfach substituierter Phenylrest ist, und wobei der Substituent
   - R: Wasserstoffatom, Methyl, Methoxy, Carboxy, Methyloxycarbonyl, CN, Hydroxy, Nitro, Fluor, Chlor, Brom, Sulfonyl, Sulfonamid oder Sulfon-(C₁-C₄)-Alkylamid ist und
   - p: 0 oder 1 ist,
   - n: 1 bis 4 ist,
   - X: Sauerstoffatom, NH, Methylenoxy, Methylenamino oder Methylen-(C₁₋C₄)-alkylamino ist,
   - Y: Sauerstoffatom oder NH ist, und
   - Wirkstoff: ein über eine Hydroxy, Amino oder Iminogruppe verknüpftes Anthrazyklin oder dessen durch eine oder mehrere Hydroxy-, Amino- oder Imino-gruppen zusätzlich substituiertes Derivat bedeutet,
   und/oder ein physiologisch verträgliches Salz der Verbindung der Formel I,
2) Zucker und/oder Zuckeralkohol ausgewählt aus Glucose, Mannose, Fructose, Galaktose, Ribose, Erythrose, Glycerinaldehyd, Sedoheptulose, Glucosamin, Galaktosamin, Glucuronsäure, Galakturonsäure, Gluconsäure, Galactonsäure, Mannonsäure, Glucitol, Mannitol (Mannit), Sorbitol, Glycerol oder Inositol;
3) zweiwertige Metallionen von Ca, Mg, Fe, Cu oder Ni, und
4) einen pharmazeutisch verträglichen Träger.

Verbindungen der Formel (I) und/oder der Formel (II) sind in EP 0 751 144 A1, EP 0 595 133 A2, EP 0 511 917 A1, EP 0 642 799 A1, Cancer Research 54 (7), 2151-2159 (1994), Anticancer Drug Design 9 (5), 409-423 (1994), Advanced Drug Delivery Reviews 19(2), 241-273 (1996) beschrieben.
Ähnliche Verbindungen mit einem anderen Spacer sind in Tetrahedron Letters 36 (10), 1701-1704 (1995), Biochem. Pharmacology 52(3), 455-463 (1996) und ohne Glycosyl Rest in EP 0 540 859 A1 beschrieben. Je nach Wirkstoff wird darin die mögliche Anwendbarkeit dieser Prodrugs für die Therapie von Krankheiten, insbesondere von Krebs, untersucht. Keines dieser Dokumente beschreibt eine Zubereitung gemäß der vorliegenden Erfindung.

Bevorzugte Anthrazykline sind Doxorubicin, 4'-epi-Doxorubicin, 4- oder 4'-Desoxydoxorubicin.

Unter dem Begriff Zucker werden Aldosen mit 3 bis 7 Kohlenstoffatomen verstanden, die der D- oder L-Reihe angehören können; dazu gehören auch Aminozucker oder Uronsäuren. Beispielhaft seien genannt Glucose, Mannose, Fructose, Galaktose, Ribose, Erythrose, Glycerinaldehyd, Sedoheptulose, Glucosamin, Galaktosamin, Glucuronsäure, Galakturonsäure, Gluconsäure, Galactonsäure oder Mannonsäure.

Zuckeralkohole entstehen beispielsweise durch Reduktion der obengenannten Zucker; dazu gehören Glucitol, Mannitol (Mannit), Sorbitol, Glycerol oder Inositol.

Geeignete physiologisch verträgliche Salze der Verbindung der Formel I sind beispielsweise Alkali-, Erdalkali- und Ammoniumsalze einschließlich solcher von organischen Ammoniumbasen und Salzen der protonierten Aminosäurereste. Bevorzugt sind Alkalisalze wie Natrium- oder Kaliumsalze.

Insbesondere enthalten die erfindungsgemäßen Zubereitungen zusätzlich zweiwertige Ionen. Unter dem Begriff "zweiwertige Ionen" werden beispielsweise zweiwertige Metallionen von Ca, Mg, Fe, Cu oder Ni verstanden.

Besonders bevorzugt werden Verbindungen der Formel I eingesetzt, worin Glykosyl ein Poly-, Oligo- oder Monosaccharid ist, insbesondere ein alpha- oder beta-O-glycosidisch verknüpfter D-Glucuronyl-, D-Glucopyranosyl-, D-Galactopyranosyl-, N-Acetyl-D-glucosaminyl, N-Acetyl-D-galactosaminyl-, D-Mannopyranosyl- oder L-Fucopyranosylrest,
- W: ein Phenylrest oder ein monosubstituierter Phenylrest ist, wobei der Substituent
- R: Methoxy, Methyloxycarbonyl, CN, Hydroxy, Nitro, Fluor, Chlor, Brom, Sulfonyl oder Sulfonamid ist und die übrigen Wasserstoffatom sind,
- X: O, NH, Methylenoxy, Methylenamino oder Methylenmethylamino ist und
- Y: O oder NH ist und
- Wirkstoff: eine Verbindung wie oben beschrieben bedeutet.

Bevorzugt werden Verbindungen eingesetzt, die dadurch gekennzeichnet sind, daß der Glykosylrest durch enzymatische Hydrolyse abgespalten werden kann, daß der Spacer durch chemische Hydrolyse spontan abgespalten werden kann, dass der Wirkstoff ein Anthrazyklin oder eines seiner durch Einführung zusätzlicher Hydroxy-, Amino- oder Iminogruppen erhaltenen Derivate ist, daß der Wirkstoff Doxorubicin, Daunomycin, Idarubicin, Epirubicin ist, und außerdem Verbindung, dadurch gekennzeichnet, daß der Glykosylrest ein alpha- oder beta-O-gyclosidisch verknüpfter D-Glucuronyl-, D-Glucopyranosyl-, D-Galactopyranosyl, N-Acetyl-D-glucosaminyl, N-Acetyl-D-galactosaminyl-, D-Mannopyranosyl- oder L-Fucopyranosylrest ist, daß sie
N-[4-O-(beta-D-Glucopyranosyluronsäure)-3-nitro-benzyloxycarbonyl]-doxorubicin Natriumsalz (Verbindung II),
N-[4-O-(beta-D-Glucopyranosyluronsäure)-3-chloro-benzyloxycarbonyl]-doxorubicin Natriumsalz,
N-[4-O-(beta-D-Glucopyranosyluronsäure)-3-fluoro-benzyloxycarbonyl]-doxorubicin Natriumsalz,
N-[4-O-(beta-D-Glucopyranosyluronsäure)-3-nitro-benzyloxycarbonyl]-daunorubicin Natriumsalz,
N-[4-O-(beta-D-Glucopyranosyluronsäure)-3-chloro-benzyloxycarbonyl]daunorubicin Natriumsalz,
N-[4-O-(alpha-D-Galaktopyranosyl)-3-nitro-benzyloxycarbonyl]-daunorubicin,
N-[4-O-(alpha-D-Galaktopyranosyl)-3-chloro-benzyloxycarbonyl]daunorubicin,
N-[4-O-(alpha-D-Galaktopyranosyl)-3-fluoro-benzyloxycarbonyl]-daunorubicin,
N-[4-O-(beta-D-Glucopyranosyluronsäure)-3-nitro-benzyloxycarbonyl]-doxorubicin Natriumsalz,
N-[2-O-(beta-D-Glucopyranosyluronsäure)-5-chloro-benzyloxycarbonyl]-doxorubicin Natriumsalz,
N-[2-O-(beta-D-Glucopyranosyluronsäure)-5-fluoro-benzyloxycarbonyl]-doxorubicin Natriumsalz,
N-[2-O-(beta-D-Glucopyranosyluronsäure)-5-nitro-benzyloxycarbonyl]-daunorubicin Natriumsalz,
N-(2-O-(beta-D-Glucopyranosyluronsäure)-5-chloro-benzyloxycarbonyl]daunorubicin Natriumsalz,
N-[2-O-(alpha-D-Galaktopyranosyl)-5-nitro-benzyloxycarbonyl]-daunorubicin,
N-[2-O-(alpha-D-Galaktopyranosyl)-5-chloro-benzyloxycarbonyl]daunorubicin,
N-[2-O-(alpha-D-Galaktopyranosyl)-5-fluoro-benzyloxycarbonyl]-daunorubicin,
14-O-[4-(beta-D-Glucuronyloxy)-3-nitrobenzylaminocarbonyl]-doxorubicin, oder
3'-N-[4-N-(alpha-D-Galactosyloxycarbonyl)-4-aminobenzyloxycarbonyl]-doxorubicin, ist.

Insbesondere bevorzugt wird die Verbindung II 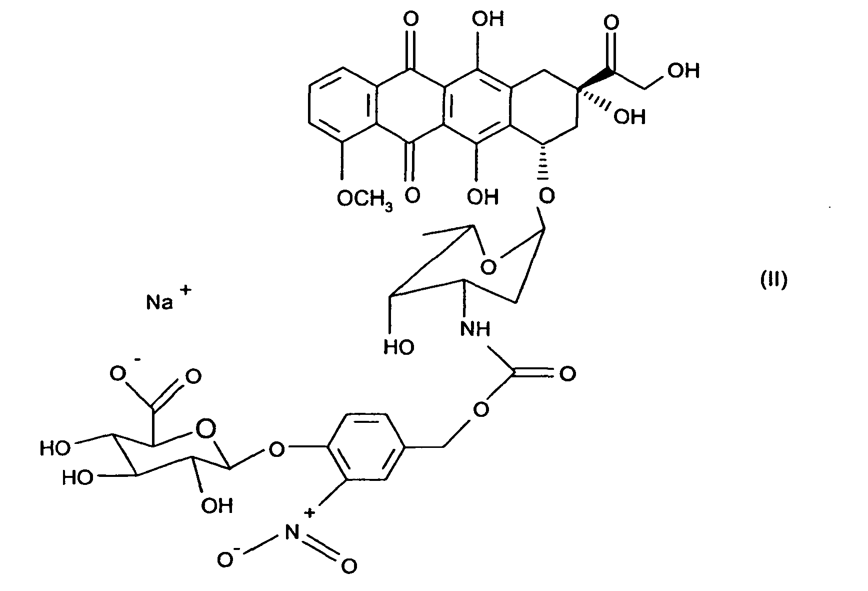 als Wirkstoff eingesetzt, sowie Mannitol als Zuckeralkohol und Ca²⁺ als zweiwertiges Ion.

Die Herstellung der Verbindung der Formel I erfolgt beispielsweise wie in EP 0 751 144 beschrieben.

Die Verbindung II wird in einer Menge von 1 bis 1000 mg/kg Lebendgewicht eingesetzt, bevorzugt von 5 bis 500 mg/kg.

Liegt eine lösliche Zubereitung vor, wird Mannitol in einer Menge von 1 mg/ml bis 150 mg/ml, bevorzugt von 10 bis 100 mg/ml, insbesondere 50 mg/ml eingesetzt. In einer löslichen Zubereitung werden Ca²⁺-Ionen, beispielsweise als CaCl₂ in einer Menge von 0,01 mg/ml bis 10 mg/ml, bevorzugt von 0,05 bis 2 mg/ml, insbesondere von 0,4 mg/ml CaCl₂ x 2 H₂O eingesetzt. Eine bevorzugte lösliche Zubereitung enthält 50 mg/ml Mannitol, 0,4 mg/ml CaCl₂x2H₂O und 25 mg/ml Verbindung (II).

Die erfindungsgemäße feste Zubereitung eignet sich beispielsweise zur Behandlung von
- akuten immunologischen Ereignissen wie Sepsis, Allergie, Graft-versus-Host- und Host-versus-Graft-Reaktionen
- Autoimmunerkrankungen, insbesondere rheumatoide Arthritis, systemischem Lupus erythematodes, multipler Sklerose
- Psoriasis, atopischer Dermatitis, Asthma, Urtikaria, Rhinitis, Uveitis
- Leberfibrose, zystischer Fibrose, Kolitis
- Krebserkrankungen wie Lungenkrebs, Leukämie, Eierstockkrebs, Sarkome, Kaposi's Sarkom, Meningiom, Darmkrebs, Lymphknotenkrebs, Himtumore, Brustkrebs, Pankreaskrebs, Prostatakrebs oder Hautkrebs.

Die erfindungsgemäße feste Zubereitung kann auch Kombinationspackungen oder Kompositionen umfassen, in denen die Bestandteile nebeneinandergestellt sind und deshalb gleichzeitig, getrennt oder zeitlich abgestuft an ein und demselben menschlichen oder tierischen Körper angewendet werden können. Erfindungsgemäß können die Komponenten 1, 2 und die zweiwertigen Ionen auch in nebeneinanderliegenden, getrennten Arzneiformen vorliegen, insbesondere dann, wenn die Arzneiformen von den räumlichen Abmessungen her eine Applikation erschweren. Dies gilt besonders für die oralen Formen, da häufig bei älteren Patienten eine Abneigung gegen große Tabletten oder Kapseln vorherrscht. Zwingend ist, daß die getrennt, nebeneinander vorliegenden Arzneiformen zur zeitlich gemeinsamen Einnahme hergerichtet sind. Dabei können auch unterschiedliche Formen, beispielsweise Tablette und Kapsel, nebeneinander vorliegen.

Die Erfindung betrifft ferner ein Verfahren zur Herstellung der erfindungsgemäßen Zubereitung, das dadurch gekennzeichnet ist, daß man die Komponenten 1), 2), die zweiwertigen Ionen und einen pharmazeutischen Träger zu einer pharmazeutischen Darreichungsform verarbeitet.

Die erfindungsgemäße feste Zubereitung kann als Dosiereinheit in Form von Arzneiformen wie Kapseln (einschließlich Mikrokapseln), Tabletten (einschließlich Dragees und Pillen) oder Zäpfchen vorliegen, wobei bei Verwendung von Kapseln das Kapselmaterial die Funktion des Trägers wahrnehmen und der Inhalt z.B. als Pulver, Gel, Emulsion, Dispersion oder Lösung vorliegen kann. Besonders vorteilhaft und einfach ist es jedoch, orale (perorale) Formulierungen mit den drei Komponenten 1), 2) und den zweiwertigen Ionen herzustellen, die die berechneten Mengen der Wirkstoffe zusammen mit jedem gewünschten pharmazeutischen Träger enthalten. Auch eine entsprechende Formulierung (Zäpfchen) für die rektale Therapie kann angewandt werden. Ebenso ist die transdermale Applikation in Form von Salben, Cremes oder orale Applikation von Lösungen, die die erfindungsgemäße Zubereitung enthalten, möglich. Ferner können die Verbindungen der Formel I auch als Lyophilisat vorliegen, das vor Applikation mit einer Lösung enthaltend 5 % Mannitol und 0,4 mg CaCl₂×2 H₂O/ml, (pH ungefähr 7) rekonstituiert wird.
Salben, Pasten, Cremes und Puder können neben den Wirkstoffen die üblichen Trägerstoffe enthalten, z.B. tierische und pflanzliche Fette, Wachse, Paraffine, Stärke, Tragacanth, Zellulosederivate, Polyethylenglykole, Silicone, Bentonite, Talkum, Zinkoxid, Milchzucker, Kieselsäure, Aluminiumhydroxid, Kalziumsilikat und Polyamidpulver oder Gemische dieser Stoffe.

Die Tabletten, Pillen oder Granulatkörper können nach üblichen Verfahren wie Preß-, Tauch- oder Wirbelbettverfahren oder Kesseldragierung hergestellt werden und enthalten Trägermittel und andere übliche Hilfsstoffe wie Gelatine, Agarose, Stärke (z.B. Kartoffel-, Mais- oder Weizenstärke), Cellulose wie Ethylcellulose, Siliziumdioxid, verschiedene Zucker wie Milchzucker, Magnesiumcarbonat und/oder Kalziumphosphate. Die Dragierlösung besteht gewöhnlich aus Zucker und/oder Stärkesirup und enthält meistens noch Gelatine, Gummi arabicum, Polyvinylpyrrolidon, synthetische Zelluloseester, oberflächenaktive Substanzen, Weichmacher, Pigmente und ähnliche Zusätze entsprechend dem Stand der Technik. Zur Herstellung der Zubereitungen kann jedes übliche Fließregulierungs-, Schmier- oder Gleitmittel wie Magnesiumstearat und Trennmittel verwendet werden.

Die anzuwendende Dosierung ist selbstverständlich abhängig von verschiedenen Faktoren wie dem zu behandelnden Lebewesen (beispielsweise Mensch oder Tier), Alter, Gewicht, allgemeiner Gesundheitszustand, dem Schweregrad der Symptome, der zu behandelnden Erkrankung, eventuellen Begleiterkrankungen, (falls vorhanden) der Art der begleitenden Behandlung mit anderen Arzneimitteln, oder der Häufigkeit der Behandlung. Die Dosierungen werden beispielsweise einmal bis dreimal pro Woche (intravenös, i.v.) verabreicht.

Die Menge der Wirkkomponenten hängt naturgemäß von der Zahl der Einzeldosierungen und auch von der zu behandelnden Krankheit ab. Die Einzeldosierung kann auch aus mehreren, gleichzeitig verabreichten Dosiereinheiten bestehen.

### Beispiele

### Pharmakologische Prüfung

Als Versuchstiere dienen tumortragende Nacktmäuse des NMRI Anszuchtstammes mit einem Körpergewicht von 17 bis 25 g. Es werden pro Versuchsgruppe 6 bis 8 Tiere eingesetzt. Die Tiere erhalten eine i.v. Applikation der Verbindung II in gelöster Form mit physiologischer Kochsalzlösung (Dosis von Verbindung II wie in Tabelle 1 beschrieben in 0,9% NaCl), Mannitol (Dosis von Verbindung II wie in Tabelle 1 beschrieben in einer 5 %igen Mannitollösung in Wasser pH 7), und einer erfindungsgemäßen Zubereitung enthaltend Verbindung II, CaCl₂ und Mannitol (Dosis von Verbindung II wie in Tabelle 1 beschrieben in einer 5 % igen Mannitollösung mit 0,4mg CaCl₂ x 2H₂O). Die Applikation erfolgt am 1., 4. und 8. Tag. Das Gewicht der Tiere sowie das Wachstum des Lovo-Tumors wird in 3 bis 4-tägigen Abständen während des gesamten Experimentes bestimmt. Die Überlebensrate wird täglich erfaßt.Tabelle 1 zeigt die Ergebnisse.

**Tabelle 1**

| galenische Formulierung | Dosis mg/kg (3x) | Wachstumsverzögerung des Tumors T-C | | minimales T/C Verhältnis | | Mittelwert des Minimalgewichtes | | tote (%) | Tiere (Tag) |
|---|---|---|---|---|---|---|---|---|---|
| | | (200%) | (400%) | (%) | (Tag) | (%) | (Tag) | | |
| physiologische | 225 | 3.9 | 19,3 | 40 | 41 | 76 | 9 | 33 | 6,11 |
| Kochsalzlösung | 350 | n.e | n.e | 85 | 4 | 78 | 4 | 100 | 4,8 |
| Mannitol | 350 | 17,3 | 18,8 | 17 | 14 | 72 | 8 | 17 | 19 |
| Ca/Mannitol | 400 | 19,6 | 28 | 13 | 23 | 82 | 10 | 0 | |

n.e. bedeutet nicht erreicht
(T-C) 200% bedeutet: Verdopplungszeit des Tumors (Tumorvolumen) unter Behandlung mit Verbindung II in der entsprechenden Zubereitung in Tagen minus Verdopplungszeit des Tumors unter Behandlung mit der entsprechenden Zubereitung ohne Verbindung II in Tagen.
(T-C) 400% bedeutet: Vervierfachungszeit des Tumors (Tumorvolumen) unter Behandlung mit Verbindung II in der entsprechenden Zubereitung in Tagen minus Vervierfachungszeit des Tumors unter Behandlung mit der entsprechenden Zubereitung ohne Verbindung II in Tagen.
minimales T/C Verhältnis (%) bedeutet: niedrigster % Wert des Tumorwachstums der Therapiegruppe im Vergleich zur Kontrollgruppe.
minimales T/C Verhältnis (Tag) bedeutet: Tag an dem das Tumorwachstum der Therapiegruppe im Vergleich zur Kontrollgruppe am niedrigsten ist.

Durch i.v. Applikation der Verbindung II in physiologischer Kochsalzlösung mit einer Dosis von 3 x 225 mg/kg wird nur ein schwacher anti-tumoraler Effekt erzielt. (T-C) 200%: 3,9 Tage. Die Gewichtsabnahme (ein Maß für die Nebenwirkungen der Verbindung II) ist allerdings bereits relativ stark (24 % Gewichtsabnahme und 33 % tote Tiere)
Die i.v. Applikation von 3x350 mg/kg der Verbindung II in physiologischer Kochsalzlösung führt zum schnellen Tod aller Versuchtiere.
Applikation von 3 x 350 mg/kg der Verbindung II in Mannitol führt zu deutlichen antitumoralen Effekten (T-C (200%):17,3 Tage) und einer moderaten Verträglichkeit (Gewichtsabnahme 28 % und 17 % tote Tiere).
Verabreichung von 3 x 400 mg/kg der Verbindung II in Ca/Mannitol induziert starke anti-tumorale Effekte (T-C (200%): 19,6 Tage) und ist für die Versuchtiere gut verträglich (Gewichtsabnahme 18 %, keine toten Tiere).

Ähnliche vorteilhafte Beobachtungen werden in Experimenten in Macaca fascicularis Affen nach i.v. Applikation von 3 x 120 mg/kg Verbindung II in der Ca/Mannitol Lösung gemacht. Die Tiere überleben diese extrem hohe Dosis ohne gravierende Anzeichen von Nebenwirkungen. Bei einer Lösung der Verbindung II in 0,1 M Phosphatpuffer, pH 7,35, werden maximal 1 x 40 mg/kg vertragen. Diese Studien belegen, daß die Verbindung II in der erfindungsgemäßen Ca/Mannitol Lösung nicht nur wesentlich besser verträglich, sondern auch deutlich wirksamer ist.

## Patentansprüche

1. Pharmazeutische Zubereitung, enthaltend
1) eine Verbindung der Formel I,
Glykosyl-Y[-C(=Y)-X-]ₚ-W(R)ₙ-X-C(=Y)-Wirkstoff (I)
worin
Glykosyl ein enzymatisch abspaltbares Poly-, Oligo- oder Monosaccharid ist,
W ein Phenylrest oder ein mehrfach substituierter Phenylrest ist, und wobei der Substituent
R Wasserstoffatom, Methyl, Methoxy, Carboxy, Methyloxycarbonyl, CN, Hydroxy, Nitro, Fluor, Chlor, Brom, Sulfonyl, Sulfonamid oder Sulfon-(C₁-C₄)-Alkylamid ist,
p 0 oder 1 ist,
n 1 bis 4 ist,
X Sauerstoffatom, NH, Methylenoxy, Methylenamino oder Methylen-(C₁-C₄)-alkylamino ist,
Y Sauerstoffatom oder NH ist, und
Wirkstoff ein über eine Hydroxy, Amino oder Iminogruppe verknüpftes Anthrazyklin oder dessen durch eine oder mehrere Hydroxy-, Amino- oder Iminogruppen zusätzlich substituiertes Derivat bedeutet,
und/oder ein physiologisch verträgliches Salz der Verbindung der Formel I,
2) Zucker und/oder Zuckeralkohol ausgewählt aus Glucose, Mannose, Fructose, Galaktose, Ribose, Erythrose, Glycerinaldehyd, Sedoheptulose, Glucosamin, Galaktosamin, Glucuronsäure, Galakturonsäure, Gluconsäure, Galactonsäure, Mannonsäure, Glucitol, Mannitol, Sorbitol, Glycerol oder Inositol,
3) zweiwertige Metallionen von Ca, Mg, Fe, Cu oder Ni, und
4) einen pharmazeutisch verträglichen Träger.

2. Zubereitung gemäß Anspruch 1, **dadurch gekennzeichnet, daß** als Anthrazyklin Doxorubicin, 4'-epi-Doxorubicin, 4- oder 4'-Desoxydoxorubicin verwendet wird.

3. Zubereitung gemäß Anspruch 1 oder 2, **dadurch gekennzeichnet, dass** als Verbindung der Formel (I) eine Verbindung der Formel (II) 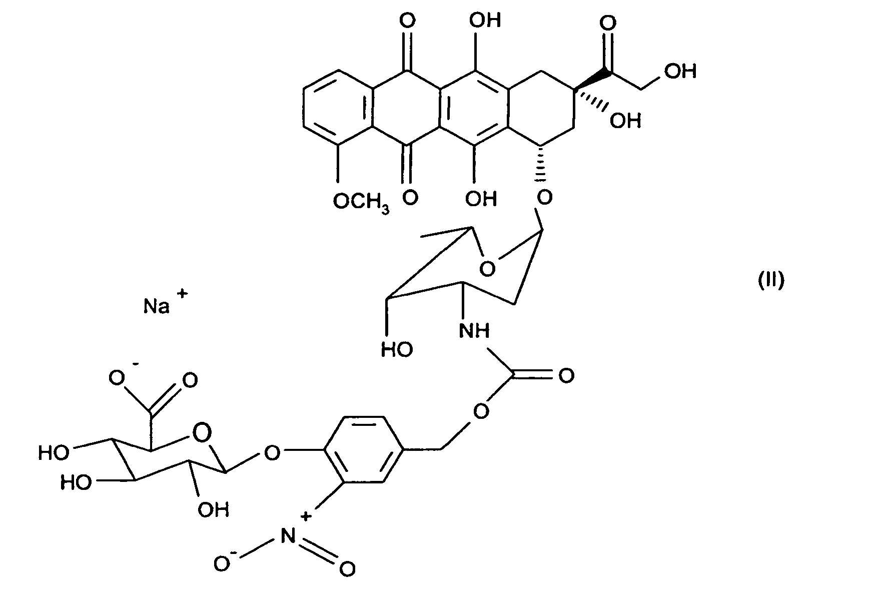 verwendet wird.

4. Zubereitung gemäß einem oder mehreren der Ansprüche 1 bis 3, **dadurch gekennzeichnet, daß** die Zubereitung als Lösung vorliegt und Ca²⁺⁻Ionen, Mannitol und Verbindung II 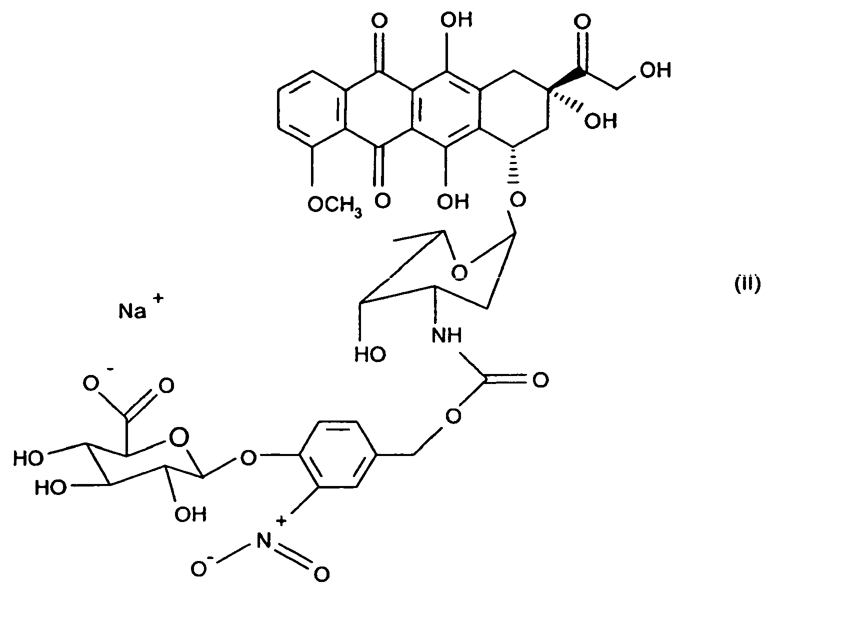 enthält.

5. Zubereitung gemäß Anspruch 4, **dadurch gekennzeichnet, daß** die Zubereitung 50 mg/ml Mannitol, 0,4 mg/ml CaCl₂ x 2H₂O und 25 mg/ml Verbindung II gemäß Anspruch 4 enthält.

6. Verfahren zur Herstellung der Zubereitung gemäß einem oder mehreren der Ansprüche 1 bis 5, **dadurch gekennzeichnet, daß** man die Verbindung der Formel I, Zucker und/oder Zuckeralkohol, zweiwertige Ionen und einen pharmazeutischen Träger zu einer pharmazeutischen Darreichungsform verarbeitet.

## Claims

1. A pharmaceutical preparation comprising
1) a compound of the formula I
glycosyl-Y[-C(=Y)-X-]ₚ-W(R)ₙ-X-C(=Y)-active compound (I)
in which
glycosyl is an enzymatically cleavable poly-, oligo- or monosaccharide
W is a phenyl radical or a polysubstituted phenyl radical,
and where the substituent
R is a hydrogen atom, methyl, methoxy, carboxyl, methyloxycarbonyl, CN, hydroxyl, nitro, fluorine, chlorine, bromine, sulfonyl, sulfonamide or sulfon-(C₁-C₄)-alkylamide,
p is 0 or 1,
n is 1 to 4,
X is an oxygen atom, NH, methyleneoxy, methyleneamino or methylene-(C₁-C₄)-alkylamino,
Y is an oxygen atom or NH, and
active compound is an anthracycline linked via a hydroxyl, amino or imino group, or its derivative additionally substituted by one or more hydroxyl, amino or imino groups,
and/or a physiologically tolerable salt of the compound of the formula I,
2) sugar and/or sugar alcohol selected from glucose, mannose, fructose, galactose, ribose, erythrose, glyceraldehyde, sedoheptulose, glucosamine, galactosamine, glucuronic acid, galacturonic acid, gluconic acid, galactonic acid, mannonic acid, glucitol, mannitol, sorbitol, glycerol or inositol,
3) divalent metal ions of Ca, Mg, Fe, Cu or Ni, and
4) a pharmaceutically tolerable carrier.

2. A preparation as claimed in claim 1, wherein the anthracycline used is doxorubicin, 4'-epi-doxorubicin, 4- or 4'-deoxydoxorubicin.

3. A preparation as claimed in claim 1 or 2, wherein a compound of the formula (II) is used as compound of the formula (I)

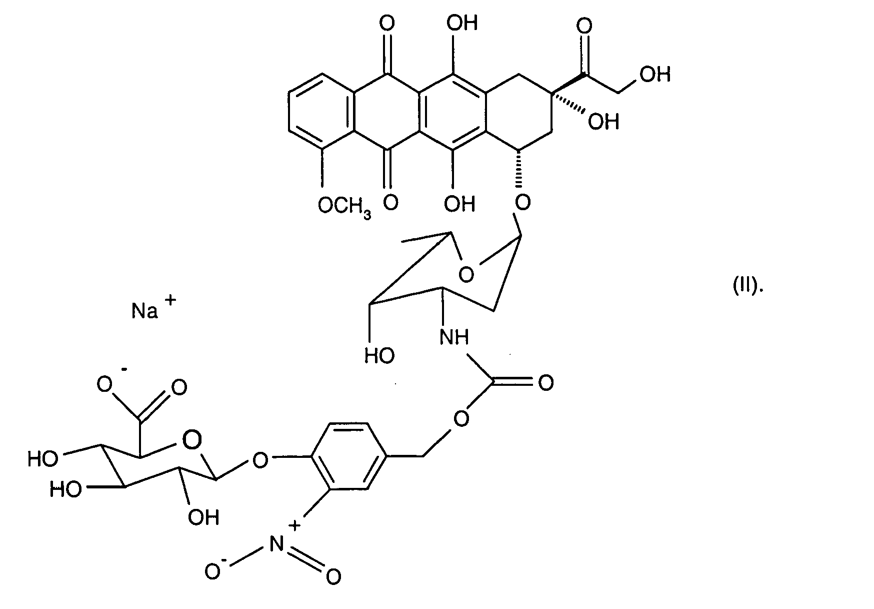

4. A preparation as claimed in one or more of claims 1 to 3, wherein the preparation is present as a solution and contains Ca²⁺ ions, mannitol and compound II

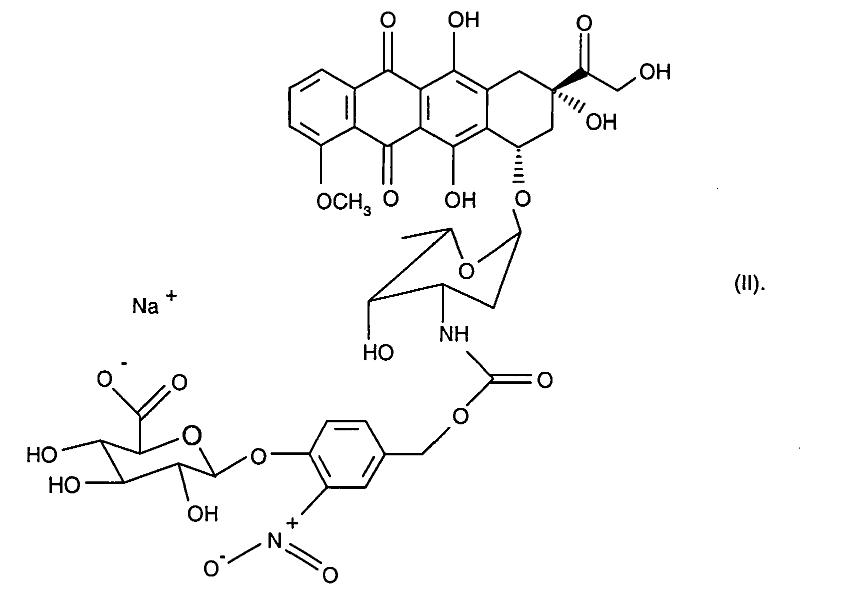

5. A preparation as claimed in claim 4, wherein the preparation contains 50 mg/ml of mannitol, 0.4 mg/ml of CaCl₂ × 2H₂O and 25 mg/ml of compound II as claimed in claim 4.

6. A process for the production of the preparation as claimed in one or more of claims 1 to 5, which comprises processing the compound of the formula I, sugar and/or sugar alcohol, divalent ions and a pharmaceutical carrier to give a pharmaceutical administration form.

## Revendications

1. Préparation pharmaceutique contenant
1) un composé de formule I,
Glycosyl-Y[-C(=Y)-X-]ₚ-W(R)ₙ-X-C(=Y)-substance active (I)
dans laquelle
Glycosyle représente un polysaccharide, un oligosaccharide ou un monosaccharide dissociable par voie enzymatique,
W représente un radical phényle ou un radical phényle polysubstitué, le substituant
R représentant un atome d'hydrogène, méthyle, méthoxy, carboxy, méthyloxycarbonyle, CN, hydroxy, nitro, fluor, chlore, brome, sulfonyle, sulfonamide ou sulfone-(alkyle en C₁ à C₄)-amide,
p vaut 0 ou 1,
n vaut 1 à 4,
X représente un atome d'oxygène, NH, méthylèneoxy, méthylèneamino ou méthylène-(alkyle en C₁ à C₄)-amino,
Y représente un atome d'oxygène ou NH, et
substance active signifie une anthracycline liée via un groupe hydroxy, amino ou imino ou son dérivé qui est en outre substitué par un ou plusieurs groupes hydroxy, amino ou imino,
et/ou un sel physiologiquement acceptable du composé de formule I,
2) un sucre et/ou un alcool de sucre choisi parmi le glucose, le mannose, le fructose, le galactose, le ribose, l'érythrose, le glycérolaldéhyde, le sédoheptulose, la glucosamine, la galactosamine, l'acide glucuronique, l'acide galacturonique, l'acide gluconique, l'acide galactonique, l'acide mannonique, le glucitol, le mannitol, le sorbitol, le glycérol ou l'inositol,
3) des ions métalliques divalents de Ca, Mg, Fe, Cu ou Ni, et
4) un support pharmaceutiquement acceptable.

2. Préparation selon la revendication 1, **caractérisée en ce qu'**on utilise comme anthracycline la doxorubicine, la 4'-épi-doxorubicine, la 4-désoxydoxorubicine ou 4'-désoxydoxorubicine.

3. Préparation selon la revendication 1 ou 2, **caractérisée en ce qu'**on utilise comme composé de formule (I) un composé de formule (II)

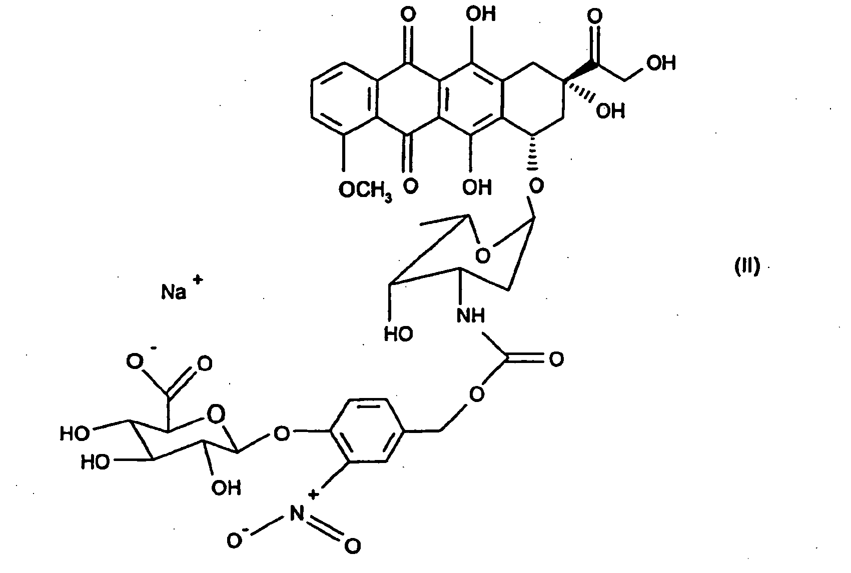

4. Préparation selon l'une ou plusieurs des revendications 1 à 3, **caractérisée en ce que** la préparation se trouve sous forme de solution et contient des ions Ca²⁺, du mannitol et le composé II

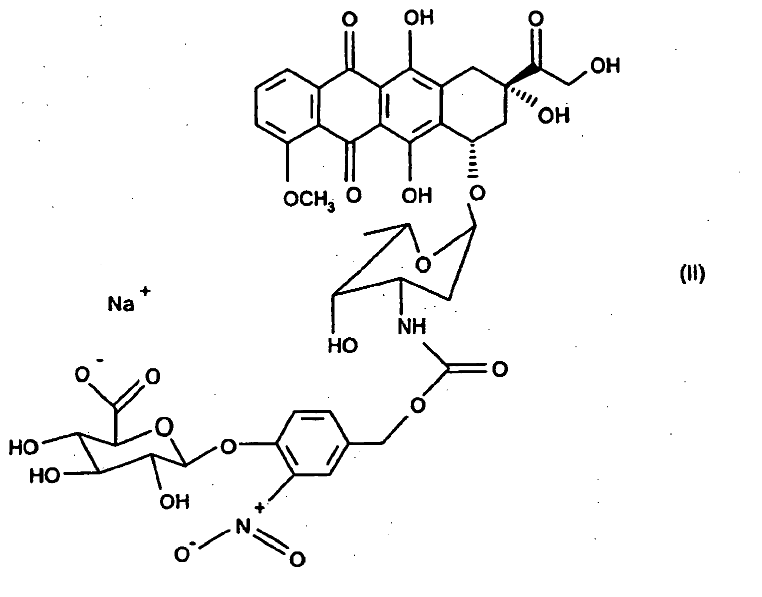

5. Préparation selon la revendication 4, **caractérisée en ce que** la préparation contient 50 mg/ml de mannitol, 0,4 mg/ml de CaCl₂ x 2H₂O et 25 mg/ml de composé II selon la revendication 4.

6. Procédé pour la préparation de la préparation selon l'une ou plusieurs des revendications 1 à 5, **caractérisé en ce qu'**on transforme le composé de formule I, le sucre et/ou l'alcool de sucre, les ions divalents et un support pharmaceutique en une forme d'administration pharmaceutique.
